# FASCICULE DE BREVET EUROPEEN

(11) **EP 4 306 936 B1**
(45) Date de publication et mention de la délivrance du brevet: **21.05.2025**
(21) Numéro de dépôt: 23306204.1
(22) Date de dépôt: 13.07.2023
(51) Int. Cl.: G01N 21/3563, G01N 33/24, G01N 21/94

(54) **DETERMINATION DE LA TENEUR EN POLLUANTS ORGANIQUES PAR SPECTROMETRIE INFRAROUGE DANS LES SOLS NATURELS ET LES MATERIAUX D'EXCAVATION**
BESTIMMUNG DES GEHALTS AN ORGANISCHEN SCHADSTOFFEN DURCH INFRAROTSPEKTROMETRIE IN NATÜRLICHEN BÖDEN UND BAGGERMATERIALIEN
DETERMINATION OF ORGANIC POLLUTANT CONTENT BY INFRARED SPECTROMETRY OF NATURAL SOIL AND EXCAVATION MATERIALS

(30) Priorité: 15.07.2022 FR 2207284
(43) Date de publication de la demande: 17.01.2024
(73) Titulaire: Eiffage GC Infra Linéaires, 78140 Vélizy-Villacoublay (FR)
(72) Inventeur: DOOM, Florian, 77144 MONTEVRAIN (FR); BOULANGE, Laurence, 38530 CHAPAREILLAN (FR)
(74) Mandataire: Plasseraud IP

(56) Documents cités:
- AU-A4- 2021 103 160
- KR-B1- 101 952 607
- US-A1- 2010 015 714
- US-A1- 2012 226 653
- US-A1- 2012 318 982
- US-A1- 2014 012 504
- US-A1- 2018 017 540
- SCAFUTTO REBECCA DEL'PAPA MOREIRA ET AL: "Quantitative characterization of crude oils and fuels in mineral substrates using reflectance spectroscopy: Implications for remote sensing", INTERNATIONAL JOURNAL OF APPLIED EARTH OBSERVATION ANDGEOINFORMATION, ELSEVIER, AMSTERDAM, NL, vol. 50, 12 April 2016 (2016-04-12), pages 221 - 242, XP029516478, ISSN: 0303-2434, DOI: 10.1016/J.JAG.2016.03.017

## Description

### Domaine technique

La présente divulgation relève du domaine de la caractérisation des sols naturels et des matériaux d'excavation, en particulier extraits par tunnelier, pour déterminer leur teneur massique en polluants organiques tels que les hydrocarbures totaux, les hydrocarbures aromatiques polycycliques, les composés organiques volatils, les polychlorobiphényles et les BTEX.

### Technique antérieure

Lors de toute construction sur ou dans le sol, en particulier lors du creusement de tunnel, l'aménagement du sol se fait par excavation. Sont alors extraites des quantités de matériau d'excavation qui dépendent de l'ampleur et du type de travaux. Typiquement, un tunnelier utilisé pour excaver un tunnel lors de la construction d'une ligne de métro produit environ 800 tonnes par jour de matériaux d'excavation.

Les matériaux d'excavation ainsi extraits contiennent des espèces chimiques variées. Certaines espèces chimiques proviennent de la composition de la roche ou du sable, on parle alors de matrice. D'autres espèces chimiques sont présentes en moins grande quantité, on parle alors de traces. Lorsque les traces présentent une toxicité, on les appelle polluants. Certains de ces polluantes sont, par exemple, des hydrocarbures. Il existe une pollution endogène, provenant de l'environnement géologique du prélèvement, et une pollution liée à l'activité humaine locale, en surface. Ces espèces chimiques peuvent représenter un risque pour l'environnement lorsque les matériaux d'excavation sont stockés après avoir été extraits. Avant de stocker ces matériaux d'excavation, il est donc nécessaire de déterminer leur taux de contamination. La détermination de ce taux de contamination permet d'orienter les matériaux dans l'une des trois filières de traitement existantes. Ces trois filières de traitement sont les suivantes :
1) Les matériaux inertes sont stockés afin d'être valorisés,
2) Les matériaux faiblement contaminés sont stockés dans des décharges spécialisées (dont le sous-sol ne permet pas d'écoulement dans des nappes phréatiques),
3) Les matériaux contaminés sont envoyés en filière de valorisation pour récupérer les éléments polluants.

La sélection de la filière de traitement adaptée dépend, entre autres, de la teneur massique en polluants organiques dans le matériau d'excavation. Par exemple, les valeurs seuils de teneur massique limite en hydrocarbures à respecter sont fixées par des textes législatifs. En France, il s'agit de la Décision n°2003/33/CE du 19.12.2002 établissant des critères et des procédures d'admission des déchets dans les décharges et des arrêtés du 30.12.2002 relatif au stockage de déchets dangereux et du 12.12.2014 relatif aux conditions d'admission des déchets inertes [...]. Selon cette Décision et ces arrêtés, les valeurs limites de teneur massique en hydrocarbures à détecter sont parmi les plus exigeantes en Europe. Ces valeurs limites sont indiquées dans le Tableau 1 ci-dessous.

**[Tableau 1]**

| Eléments polluants organiques à détecter | Valeur limite I.S.D.I.* | Valeur limite I.S.D.N.D.** | Valeur limite I.S.D.D.*** |
|---|---|---|---|
| | Teneur massique en mg/kg de matière sèche | | |
| Hydrocarbures Aromatiques Polycycliques (HAP) | 50 | 100 | 500 |
| Composés Organiques Volatils (COV) | 30 000 | 50 000 | 60 000 |
| Polychlorobiphényles (PCB) | 1 | 50 | - |
| Hydrocarbures Totaux (HCT) | 500 | 800 | 1 000 |
| Benzène, Toluène, Ethylbenzène et Xylène (BTEX) | 6 | 30 | - |

| | | | |
|---|---|---|---|
| I.S.D.I. : Installation de Stockage de Déchets Inertes I.S.D.N.D : Installation de Stockage de Déchets non Dangereux I.S.D.D : Installation de Stockage de Déchets Dangereux * Annexe II de l'arrêté du 12.12.2014 ** Décision n°2003/33/CE du 19.12.2002 *** Annexe I de l'arrêté du 30.12.2002 | | | |

Si la teneur massique de chaque élément polluant organique contenu dans le matériau d'excavation est inférieure à la valeur limite I.S.D.I. indiquée dans le Tableau 1, alors le matériau d'excavation est considéré comme inerte. Il peut alors être stocké dans des installations de stockage de déchets inertes afin d'être valorisé, par exemple en tant que matériau de construction ou pour la valorisation paysagère

Si la teneur massique d'au moins un des éléments polluants organiques contenu dans le matériau d'excavation est comprise entre les valeurs limites I.S.D.I et I.S.D.N.D. indiquées dans le Tableau 1, alors le matériau d'excavation est considéré comme faiblement contaminé. Il peut alors être stocké dans des installations de stockage de déchets non dangereux.

Si la teneur massique d'au moins un des éléments polluants organiques contenu dans le matériau d'excavation est comprise entre les valeurs limites I.S.D.N.D. et I.S.D.D. indiquées dans le Tableau 1, alors le matériau d'excavation est considéré comme contaminé. Il est alors stocké dans des installations de stockage de déchets dangereux. Il peut y être décontaminé pour récupérer et valoriser les éléments polluants organiques.

La procédure pour déterminer la teneur massique de chaque élément polluant organique dans un matériau d'excavation est fixée par des normes nationales. En France, il s'agit des normes françaises NF EN ISO 16703 pour les hydrocarbures totaux, NF EN 17503 pour les hydrocarbures aromatiques polycycliques, ISO 22155 pour les BTEX et les composés organiques volatils et NF EN 17322 pour les polychlorobiphényles.

Les procédures pour déterminer la teneur massique des hydrocarbures totaux, des hydrocarbures aromatiques polycycliques, des BTEX, des composés organiques volatils et des polychlorobiphényles présents dans un matériau d'excavation sont identiques mais sont très longues. Ces procédures consistent en une analyse physicochimique de la composition du lixiviat obtenu à l'issue d'une lixiviation simulée du matériau d'excavation. Selon les normes françaises mentionnées ci-dessus, une lixiviation (traitement par l'eau bien connu qui entraîne la dissolution des espèces solubles) du matériau d'excavation est simulée pendant 24 heures à température ambiante (20°C ± 5°C). Puis le lixiviat (liquide résiduel de la lixiviation) est analysé pour déterminer la teneur massique des hydrocarbures totaux, des hydrocarbures aromatiques polycycliques, des BTEX, des composés organiques volatils et des polychlorobiphényles présents dans ledit lixiviat. La teneur massique déterminée dans le lixiviat est représentative de la teneur massique des hydrocarbures totaux, des hydrocarbures aromatiques polycycliques, des BTEX, des composés organiques volatils et des polychlorobiphényles présents dans le matériau d'excavation.
La durée de ces procédures est au minimum 48 heures. Il faut également ajouter à cette durée le délai de transport vers un laboratoire externe au site d'extraction des matériaux d'excavation et le délai de traitement pour une durée totale générale de sept jours.
Durant cette longue durée, il est nécessaire de stocker les matériaux excavés extraits sur les chantiers qui se trouvent très souvent implantés dans des zones géographiques à très fortes contraintes (zone urbaine, vallée montagneuse).

La détection des hydrocarbures totaux est décrite dans US 2018/017540 A1, US 2010/015714 A1, US 2014/012504 A1 et US 2012/226653 A1. La détection des hydrocarbures aromatiques polycycliques est décrite dans US 2012/318982 A1 et AU 2021 103 160 A4. Rebecca Del'Papa Moreira Scafutto et al, International Journal of Applied Earth Observation and geoinformation, vol 50, 12 avril 2016, page 221-242 décrit l'utilisation de la spectroscopie pour caractériser des huiles brutes et des carburants dans des substrats minéraux.

De façon surprenante, la Demanderesse a trouvé une méthode permettant de déterminer rapidement la teneur massique de tous les éléments polluants organiques compris dans un matériau d'excavation.

### Résumé

Il est proposé une méthode d'analyse d'un matériau d'excavation comme définie dans la revendication 1.

De façon avantageuse, l'étape a) de la méthode de la présente invention permet d'obtenir un spectre infrarouge pouvant comprendre :
- des bandes d'absorptions entre 1630 cm⁻¹ et 1680 cm⁻¹ et vers 1645 cm⁻¹ qui sont spécifiques aux hydrocarbures totaux,
- des bandes d'absorption vers 1450 cm⁻¹, 1500 cm⁻¹, 1580 cm⁻¹ et 1600 cm⁻¹ qui sont spécifiques aux hydrocarbures aromatiques polycycliques,
- des bandes d'absorptions entre 3610 cm⁻¹ et 3670 cm⁻¹, entre 3200 cm⁻¹ et 3400 cm⁻¹ et vers 1735 cm⁻¹ qui sont spécifiques aux composés organiques volatils,
- des bandes d'absorption vers 540 cm⁻¹ et 760 cm⁻¹ qui sont spécifiques aux polychlorobiphényles, et
- une bande d'absorption entre 1610 cm⁻¹ et 1650 cm⁻¹ qui est spécifique aux BTEX.

Comme indiqué au Tableau 1 ci-dessus, les teneurs massiques en polluants organiques à détecter pour la valeur limite I.S.D.I. sont très faibles. De plus, il est couramment admis que la limite de détection basse doit être trois fois inférieure à la valeur limite à détecter. Il est ainsi nécessaire de pouvoir détecter une teneur massique en HAP d'au moins 16 mg/kg.
Grâce aux gammes de nombre d'onde indiquées ci-dessus, la méthode d'analyse permet de déterminer de façon fiable et reproductibles les faibles teneurs massiques en hydrocarbures totaux, en hydrocarbures aromatiques polycycliques, en composés organiques volatils, en polychlorobiphényles et en BTEX présents dans le matériau d'excavation.
En effet, ces gammes de nombre d'onde ne se recoupent pas avec la gamme 2700 cm⁻¹ - 3100 cm⁻¹ dans laquelle la bande spécifique de l'eau, élément très présent dans le matériau d'excavation, est détectable.

En déterminant les aires d'une ou plusieurs de ces bandes d'absorption et en la comparant à une courbe de calibration préalablement obtenue à partir de l'analyse par spectroscopie d'échantillons dont les teneurs massiques en hydrocarbures totaux, en hydrocarbures aromatiques polycycliques, en composés organiques volatils, en polychlorobiphényles et/ou en BTEX sont connues, il est possible de déterminer précisément, les teneurs massiques en hydrocarbures totaux, en hydrocarbures aromatiques polycycliques, en composés organiques volatils, en polychlorobiphényles et en BTEX dans l'échantillon du matériau d'excavation.

De façon avantageuse, la méthode de la présente invention permet de déterminer simplement, rapidement et de façon fiable et reproductibles les teneurs massiques en hydrocarbures totaux, en hydrocarbures aromatiques polycycliques, en composés organiques volatils, en polychlorobiphényles et en BTEX présents dans le matériau d'excavation.

En effet, les spectromètres infrarouge sont des appareils connus, compacts, fiables et robustes qui peuvent être utilisés sur un chantier et qui peuvent réaliser l'étape a) d'analyse en quelques secondes.

De plus les teneurs massiques en hydrocarbures totaux, en hydrocarbures aromatiques polycycliques, en composés organiques volatils, en polychlorobiphényles et en BTEX présents dans le matériau d'excavation déterminées par la méthode d'analyse de la présente invention sont en adéquation avec les teneurs massiques en hydrocarbures totaux, en hydrocarbures aromatiques polycycliques, en composés organiques volatils, en polychlorobiphényles et en BTEX présents dans le matériau d'excavation déterminées par la procédure conventionnelle selon les normes en vigueur.

Il est également proposé une méthode de sélection d'une filière de traitement des matériaux d'excavation comprenant les étapes suivantes :
c) comparaison de la teneur massique de chaque polluant organique déterminée par la méthode d'analyse telle que décrite ci-dessus avec une valeur limite fixée,
d) sélection de la filière de traitement selon les critères suivants:
   - si la teneur massique de chaque polluant organique contenu dans l'échantillon est inférieure à la valeur limite fixée alors le matériau est considéré comme inerte (Filière 1),
   - si la teneur massique d'un polluant organique est supérieure à la valeur limite fixée alors le matériau est considéré comme contaminé et peut être stocké dans des décharges spécialisées (Filière 2) ou peut être décontaminé (Filière 3).

La valeur limite fixée pour chacun des éléments polluants organiques peut être fixée par un texte législatif ou réglementaire national ou international. Cette valeur limite fixée peut être celle indiquée dans le Tableau 1 ci-dessus.

### Brève description des dessins

D'autres caractéristiques, détails et avantages apparaîtront à la lecture de la description détaillée ci-après, et à l'analyse des dessins annexés, sur lesquels :
**Fig. 1**

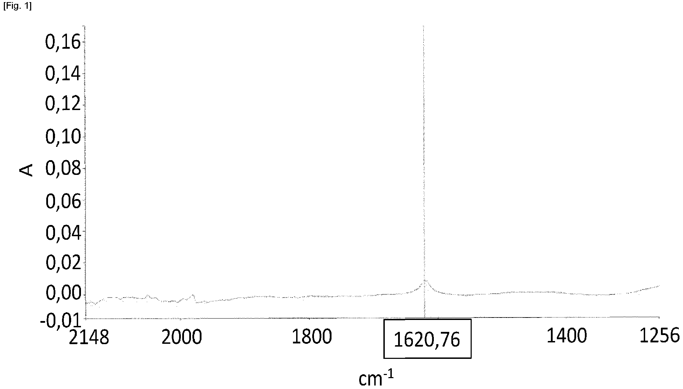

[Fig. 1] montre un spectre infrarouge d'un échantillon de matériau d'excavation subissant la méthode d'analyse selon l'invention.

### Description des modes de réalisation

Selon un premier objet de l'invention, il est proposé une méthode d'analyse d'un matériau d'excavation comprenant les étapes suivantes :
a) analyse par spectroscopie infrarouge d'un échantillon d'un matériau d'excavation dans une gamme de nombres d'onde comprise entre 4000 cm⁻¹ et 500 cm⁻¹, en particulier entre 3500 cm⁻¹ et 600 cm⁻¹, plus particulièrement entre 3000 cm⁻¹ et 650 cm⁻¹ pour obtenir un spectre infrarouge,
b) détermination de la teneur massique en polluants organiques dans ledit échantillon à partir du spectre infrarouge obtenu à l'étape a),
les polluants organiques étant choisis parmi les hydrocarbures totaux, les hydrocarbures aromatiques polycycliques, les composés organiques volatils, les polychlorobiphényles, les BTEX et leurs combinaisons.

Au sens de la présente demande, "spectroscopie infrarouge" désigne une technique d'analyse chimique reposant sur l'absorption de la lumière par la plupart des molécules dans la région de l'infrarouge du spectre électromagnétique et en convertissant cette absorption en vibration moléculaire. Cette absorption correspond spécifiquement aux liaisons présentes dans la molécule. Avec un spectromètre, cette absorption du rayonnement infrarouge par le matériau de l'échantillon est mesurée en fonction des nombres d'onde. Le résultat est un spectre infrarouge comprenant une ou des bandes d'absorption spécifique(s) aux molécules présentes dans l'échantillon analysé. La bande d'absorption peut également être appelée pic dans la présente demande.

Au sens de la présente demande, "matériau d'excavation" désigne tout matériau excavé lors de travaux de génie civil ou de construction que ce soit à la surface de la Terre, par exemple lors de fouilles ou de création de fondation, ou dans le sous-sol, par exemple lors du creusement de tunnels, cavernes et galeries. Typiquement, le matériau d'excavation comprend des :
- roches meubles tels que graviers, sables, limons, argiles et leurs mélanges ;
- rochers concassés ;
- matériaux provenant de constructions antérieures ou de sites pollués tels que des décharges ; ou
- boues d'excavation.

Selon un mode de réalisation, le matériau d'excavation est une boue d'excavation.

Typiquement, la boue d'excavation peut être produite par un tunnelier excavant un tunnel lors de la construction d'une ligne de métro, d'une ligne de train, d'une route.

Selon un mode de réalisation particulier, la boue d'excavation est extraite du sous-sol parisien.

Dans la présente demande, les polluants organiques sont choisis parmi les hydrocarbures totaux, les hydrocarbures aromatiques polycycliques, les composés organiques volatils, les polychlorobiphényles, les BTEX et leurs combinaisons.

Au sens de la présente demande, "hydrocarbures totaux", aussi noté HCT, désigne un ensemble de composés organiques provenant de la distillation du pétrole et faisant partie de la famille des polluants organiques persistants dont la chaine comprend entre 10 et 40 atomes de carbone. Ces composés peuvent être des n-alcanes, des iso-alcanes, des cyclo-alcanes, les alkylbenzènes et/ou des alkylnaphthalènes.

Au sens de la présente demande, "hydrocarbures aromatiques polycycliques", aussi noté HAP, désigne un ensemble de composés organiques constitués d'atomes de carbone et d'hydrogène et dont la structure comprend au moins deux cycles aromatiques condensés. Le naphtalène, l'acénaphtylène, l'acénaphtène, le fluorène, le phénanthrène, l'anthracène, le fluoranthène, le pyrène, le benzo(a)anthracène, le chrysène, le bbenzo(b)fluoranthène, le benzo(k)fluoranthène, le benzopyrène (BaP), l'indeno(123) pyrène et le dibenzo(a,h)anthracène font partie de cet ensemble.

Au sens de la présente demande, "composés organiques volatils", aussi noté COV, désigne des composés organiques se trouvant sous forme gazeuse dans l'atmosphère terrestre.

Au sens de la présente demande, "polychlorobiphényles", aussi noté ou PCB, désigne des hydrocarbures halogénés, des composés aromatiques organochlorés de haut poids moléculaire, dérivés du biphényle.

Au sens de la présente demande, "BTEX" désigne un groupe de composés organiques qui comprend le benzène, le toluène, l'éthylbenzène et les xylènes (m-p-xylènes et o-xylène).

Le matériau d'excavation mis en œuvre dans l'étape a) peut présenter un taux de matière sèche, ou siccité, de 70% à 100%, en particulier de 90% à 100%, tout particulièrement de 99% à 99,99%.

Alternativement, le matériau d'excavation mis en œuvre dans l'étape a) peut présenter un taux de matière sèche, ou siccité, de 70% à 100%, en particulier de 75% à 90%, tout particulièrement de 78% à 82%.

Au sens de la présente demande, le "taux de matière sèche" est le rapport entre la masse sèche de matériau d'excavation et la masse du matériau d'excavation avant séchage, la masse sèche du matériau d'excavation étant mesurée après séchage d'environ 30 grammes de matériau d'excavation pendant 30 minutes à 145°C.

De façon avantageuse, mettre en œuvre un matériau d'excavation présentant un tel taux de matière sèche permet d'obtenir un spectre infrarouge dont la résolution permet l'identification des bandes d'absorption spécifiques aux polluants organiques.

Le séchage d'un matériau d'excavation est une étape classique connue de l'homme du métier. Il saura donc mettre en œuvre une étape de séchage du matériau d'excavation pour obtenir le taux de matière sèche décrit ci-dessus.

Le matériau d'excavation mis en œuvre dans l'étape a) peut présenter une granulométrie telle que 35% massique du matériau d'excavation présente un diamètre inférieur à 63 µm, en particulier 95% massique du matériau d'excavation présente un diamètre inférieur à 125 µm, tout particulièrement 100% massique du matériau d'excavation présente un diamètre inférieur à 200°µm.

La granulométrie du matériau d'excavation peut être déterminée par tamisage.

Cette granulométrie peut être obtenue par un broyage manuel ou un broyage automatique, en particulier un broyage manuel, plus particulièrement un broyage manuel dans un moule en agate. Le broyage manuel est avantageusement adapté à la disparité de dureté des composants du matériau d'excavation.

Selon l'invention, l'étape b) de la méthode d'analyse comprend les sous-étapes suivantes :
b11) détection entre 1600 cm⁻¹ et 1700 cm⁻¹ d'une bande d'absorption spécifique aux hydrocarbures totaux dans le spectre infrarouge obtenu à l'étape a),
b21) intégration de la bande d'absorption détectée à l'étape b11) pour calculer une aire, et
b31) comparaison de l'aire obtenue à l'étape b21) avec une courbe de calibration pour déterminer la teneur massique en hydrocarbures totaux du matériau d'excavation.

De façon avantageuse, la gamme de nombres d'onde de la sous-étape b11) permet de calculer, de façon reproductible et précise, l'aire de la bande d'absorption spécifique aux hydrocarbures totaux lors de l'étape b21). A partir de cette aire, il est ensuite possible de déterminer, de façon reproductible et précise, la teneur massique en hydrocarbures totaux du matériau d'excavation lors de l'étape b31).

Ces sous-étapes b11) à b31) peuvent permettre de déterminer rapidement, précisément et de façon reproductible, la teneur massique en hydrocarbures totaux du matériau d'excavation.

L'étape b) de la méthode d'analyse peut, alternativement ou additionnellement aux sous-étapes b11) à b31) comprendre les sous-étapes suivantes :
b12) détection entre 1400 cm⁻¹ et 1500 cm⁻¹ d'une bande d'absorption spécifique aux hydrocarbures aromatiques polycycliques dans le spectre infrarouge obtenu à l'étape a),
b22) intégration de la bande d'absorption détectée à l'étape b12) pour calculer une aire, et
b32) comparaison de l'aire obtenue à l'étape b22) avec une courbe de calibration pour déterminer la teneur massique en hydrocarbures aromatiques polycycliques du matériau d'excavation.

De façon avantageuse, la gamme de nombres d'onde de la sous-étape b12) permet de calculer, de façon reproductible et précise, l'aire de la bande d'absorption spécifique aux hydrocarbures aromatiques polycycliques lors de l'étape b22). A partir cette aire, il est ensuite possible de déterminer, de façon reproductible et précise, la teneur massique en hydrocarbures aromatiques polycycliques du matériau d'excavation lors de l'étape b32).

Ces sous-étapes b12) à b32) peuvent permettre de déterminer rapidement, précisément et de façon reproductible, la teneur massique en hydrocarbures aromatiques polycycliques du matériau d'excavation.

L'étape b) de la méthode d'analyse peut, alternativement ou additionnellement aux sous-étapes b11) à b31) et b12) à b32), comprendre les sous-étapes suivantes :
b13) détection entre 3300 cm⁻¹ et 3400 cm⁻¹ d'une bande d'absorption spécifique aux composés organiques volatils dans le spectre infrarouge obtenu à l'étape a),
b23) intégration de la bande d'absorption détectée à l'étape b13) pour calculer une aire, et
b33) comparaison de l'aire obtenue à l'étape b23) avec une courbe de calibration pour déterminer la teneur massique en composés organiques volatils du matériau d'excavation.

De façon avantageuse, la gamme de nombres d'onde de la sous-étape b13) permet de calculer, de façon reproductible et précise, l'aire de la bande d'absorption spécifique aux composés organiques volatils lors de l'étape b23). A partir cette aire, il est ensuite possible de déterminer, de façon reproductible et précise, la teneur massique en composés organiques volatils du matériau d'excavation lors de l'étape b33).

Ces sous-étapes b13) à b33) peuvent permettre de déterminer rapidement, précisément et de façon reproductible, la teneur massique en composés organiques volatils du matériau d'excavation.

L'étape b) de la méthode d'analyse peut, alternativement ou additionnellement aux sous-étapes b11) à b31), b12) à b32) et b13) à b33) comprendre les sous-étapes suivantes :
b14) détection entre 800 cm⁻¹ et 500 cm⁻¹ d'une ou de deux bandes d'absorption spécifiques aux polychlorobiphényles dans le spectre infrarouge obtenu à l'étape a),
b24) intégration des bandes d'absorption détectées à l'étape b14) pour calculer une aire, et
b34) comparaison de l'aire obtenue à l'étape b24) avec une courbe de calibration pour déterminer la teneur massique en polychlorobiphényles du matériau d'excavation.

De façon avantageuse, la gamme de nombres d'onde de la sous-étape b14) permet de calculer, de façon reproductible et précise, l'aire des bandes d'absorption spécifiques aux polychlorobiphényles lors de l'étape b24). A partir cette aire, il est ensuite possible de déterminer, de façon reproductible et précise, la teneur massique en polychlorobiphényles du matériau d'excavation lors de l'étape b34).

Ces sous-étapes b14) à b34) peuvent permettre de déterminer rapidement, précisément et de façon reproductible, la teneur massique en polychlorobiphényles du matériau d'excavation.

L'étape b) de la méthode d'analyse peut, alternativement ou additionnellement aux sous-étapes b11) à b31), b12) à b32), b13) à b33) et b14) à b34) comprendre les sous-étapes suivantes :
b15) détection entre 1600 cm⁻¹ et 1650 cm⁻¹ d'une bande d'absorption spécifique aux BTEX dans le spectre infrarouge obtenu à l'étape a),
b25) intégration de la bande d'absorption détectée à l'étape b15) pour calculer une aire, et
b35) comparaison de l'aire obtenue à l'étape b25) avec une courbe de calibration pour déterminer la teneur massique en BTEX du matériau d'excavation.

De façon avantageuse, la gamme de nombres d'onde de la sous-étape b15) permet de calculer, de façon reproductible et précise, l'aire de la bande d'absorption spécifique aux BTEX lors de l'étape b25). A partir cette aire, il est ensuite possible de déterminer, de façon reproductible et précise, la teneur massique en BTEX du matériau d'excavation lors de l'étape b35).

Ces sous-étapes b15) à b35) peuvent permettre de déterminer rapidement, précisément et de façon reproductible, la teneur massique en BTEX du matériau d'excavation.

Grâce à la détermination précise des teneurs massiques en hydrocarbures totaux, en hydrocarbures aromatiques polycycliques, en composés organiques volatils, en polychlorobiphényles et en BTEX du matériau d'excavation par la méthode d'analyse de la présente invention, il est possible de sélectionner la bonne filière de retraitement d'un matériau d'excavation.

Ainsi, selon un deuxième objet de l'invention, il est proposé une méthode de sélection d'une filière de retraitement de matériaux d'excavation comprenant les étapes suivantes:
c) comparaison de la teneur massique de chaque polluant organique déterminée par la méthode d'analyse telle que décrite ci-dessus avec une valeur limite fixée,
d) sélection de la filière de traitement selon les critères suivants:
   - si la teneur massique de chaque polluant organique contenu dans l'échantillon est inférieure à la valeur limite fixée alors le matériau est considéré comme inerte (Filière 1),
   - si la teneur massique d'un polluant organique est supérieure à la valeur limite fixée alors le matériau est considéré comme contaminé et peut être stocké dans des décharges spécialisées (Filière 2) ou peut être décontaminé (Filière 3).

Dans cette méthode de sélection, les polluants organiques sont les mêmes que les polluants organiques de la méthode d'analyse, i.e. ceux choisis parmi les hydrocarbures totaux, les hydrocarbures aromatiques polycycliques, les composés organiques volatils, les polychlorobiphényles, les BTEX et leurs combinaisons.

Typiquement, selon la législation française, le matériau d'excavation est considéré comme inerte si la teneur massique de chaque polluant organique est inférieure à la valeur limite valeur limite I.S.D.I. indiquées dans le Tableau 1. Ce matériau d'excavation est alors réutilisable, par exemple en tant que matériau de construction ou pour la valorisation paysagère (Filière 1).

Typiquement, selon la législation française, le matériau d'excavation est considéré comme faiblement contaminé si la teneur massique d'un polluant organique contenu dans ledit matériau d'excavation est comprise entre les valeurs limites I.S.D.I et I.S.D.N.D. indiquées dans le Tableau 1. Ce matériau d'excavation considéré comme faiblement contaminé est stocké dans des décharges spécialisées telles que des installations de stockage de déchets non dangereux (Filière 2).

Typiquement, selon la législation française, le matériau d'excavation est considéré comme contaminé si la teneur massique d'un polluant organique contenu dans ledit matériau d'excavation est comprise entre les valeurs limites I.S.D.N.D. et I.S.D.D. indiquées dans le Tableau 1. Ce matériau d'excavation considéré comme contaminé est stocké dans des décharges spécialisées telles que des installations de stockage de déchets dangereux. Typiquement il peut y être décontaminé pour récupérer et valoriser les éléments polluants (Filière 3a).

Typiquement, le matériau d'excavation est considéré comme fortement contaminé si la teneur massique d'un polluant organique contenu dans ledit matériau d'excavation est supérieure à la valeur limite I.S.D.D. indiquée dans le Tableau 1. Ce matériau d'excavation considéré comme fortement contaminé est stocké dans des décharges spécialisées. Typiquement il peut y être décontaminé pour récupérer et valoriser les éléments polluants (Filière 3b).

Un troisième objet de l'invention est une méthode de valorisation d'un matériau d'excavation considéré comme inerte selon la méthode de sélection du deuxième objet de l'invention telle que définie ci-dessus, ladite méthode comprenant une étape de valorisation dudit matériau d'excavation en tant que matériau de construction.

Typiquement, le matériau de construction peut être un remblai ou un granulat, en particulier un remblai, un granulat pour béton ou un granulat pour enrobé.

Au sens de la présente demande, "remblai" désigne un matériau de construction destiné à élever un terrain, combler un creux ou combler les vides de l'exploitation minière.

Au sens de la présente demande, "granulat" désigne un matériau de construction utilisé pour la réalisation d'ouvrages de Génie Civil, de travaux routiers et de bâtiments.

Un granulat pour béton et un granulat pour enrobé sont des exemples de granulat.

Au sens de la présente demande, "granulat pour enrobé" désigne un granulat utilisé pour la confection des enrobés bitumineux.

Au sens de la présente demande, "granulat pour béton" désigne un granulat utilisé pour la confection des bétons.

L'étape de valorisation peut comprendre une étape de production d'un matériau de construction à partir du matériau inerté.

Typiquement l'étape de production peut comprendre une ou des sous-étapes de mise en forme du matériau inerté, la ou les sous-étapes étant adaptées au matériau de construction, en particulier au remblai ou au granulat, plus particulièrement au remblai, au granulat pour béton ou au granulat pour enrobé.

Le choix du matériau de construction dépend de la concentration en éléments polluants organiques compris dans le matériau d'excavation. Ainsi l'étape de production, la ou les sous-étapes de mise en forme du matériau inerté peuvent être choisies grâce à la méthode de détermination telle que définie ci-dessus.

Un quatrième objet de l'invention est une méthode de stockage d'un matériau d'excavation considéré comme contaminé selon la méthode de sélection du deuxième objet de l'invention telle que définie ci-dessus, ladite méthode comprenant une étape de stockage dudit matériau d'excavation.

En fonction de la teneur massique des polluants organiques contenus dans le matériau d'excavation, le matériau est stocké dans une Installation de Stockage de Déchets non Dangereux ou dans une Installation de Stockage de Déchets Dangereux. L'installation de stockage peut être choisie grâce à la méthode d'analyse telle que définie ci-dessus.

L'étape de stockage dépend de l'installation dans lequel le matériau inerté est stocké. Ainsi l'étape de stockage peut être adaptée à un stockage dans une Installation de Stockage de Déchets non Dangereux ou dans une Installation de Stockage de Déchets Dangereux, en particulier dans une Installation de Stockage de Déchets Dangereux.

Le stockage de matériau dans une de ces installations est connu de l'homme du métier. Il saura donc mettre en œuvre l'étape de stockage.

Un cinquième objet de l'invention est une méthode de décontamination d'un matériau d'excavation considéré comme contaminé selon la méthode de sélection du deuxième objet de l'invention telle que définie ci-dessus, ladite méthode comprenant une étape de décontamination dudit matériau d'excavation.

La décontamination d'un matériau d'excavation est une étape connue de l'homme du métier. Il saura donc la mettre en œuvre.

### Exemples

### Exemple 1 : Détermination de la teneur massique des HCT, des HAP et des COV de différents échantillons de sol.

Différents échantillons de sol sont testés après une phase préalable de préparation qui consiste en un séchage à 145°C pendant 30 minutes, suivi d'un léger broyage puis d'un tamisage à 200 µm. Les échantillons de sol ainsi préparés présentent un taux de matière sèche d'environ 99,99%.

Des analyses conventionnelles selon les normes en vigueur ont permis de déterminer la teneur massique des HCT, des HAP et des COV dans les différents échantillons de sols. L'analyse rapide par IR (de 400 cm⁻¹ à 4000 cm⁻¹, Interféromètre Michelson de résolution 0.5 cm⁻¹ et détecteur DTGS) a permis de détecter les HCT au pic spécifique de 1640 cm⁻¹, les HAP au pic spécifique de 1400 cm⁻¹ et les COV au pic spécifique de 3350 cm⁻¹. Les résultats sont présentés dans le Tableau 2 ci-dessous.

Malgré le séchage quasi-complet des échantillons de sol, une bande spécifique de l'eau est détectée entre 2700 cm⁻¹ et 3100 cm⁻¹. Cette bande empêche toute détection d'un pic spécifique des polluants organiques dont les teneurs massiques indiquées dans le Tableau 2 ci-dessous sont très faibles.

**[Tableau 2]**

| **Polluants organiques** | **Ech. 1** | **Ech. 2** | **Ech. 3** | **Ech.4** |
|---|---|---|---|---|
| Teneur massique en HCT | < 20 | 170 | 310 | 850 |
| (mg/Kg de matières sèche) | | | | |
| déterminée selon norme NF EN ISO 16703 | | | | |
| Aire du pic à 1640 cm⁻¹ | 1 | 2,5 | 2,2 | 1,3 |
| Teneur massique en HAP (mg/Kg de matières sèche) déterminée selon norme NF EN 17503 | - | 35 | 59 | 170 |
| Aire du pic à 1450 cm⁻¹ | -. | 3,6 | 7,3 | 10,5 |
| Teneur massique en COV (mg/Kg de matières sèche) selon norme ISO 22155 | - | - | - | 39000 |
| Aire du pic à 3350 cm⁻¹ | - | - | - | 32,2 |

Le Tableau 2 met en évidence qu'il est possible de détecter rapidement la présence des HCT, des HAP et des COV dans des sols grâce à une analyse par spectroscopie infrarouge d'échantillon de ces sols.
De plus les aires des pics calculées et présentées dans le Tableau 2 sont en accord avec les teneurs massiques des HCT, des HAP et des COV dans les sols déterminées par des analyses conventionnelles selon les normes en vigueur.

### Exemple 2 : Détermination de la teneur massique des BTEX dans un échantillon de sol.

Un échantillon de sol est testé après une phase préalable de préparation qui consiste en un séchage à 145°C pendant 30 minutes, suivi d'un léger broyage puis d'un tamisage à 200 µm. L'échantillon de sol ainsi préparé présente un taux de matière sèche d'environ 99,99%.
Une analyse conventionnelle selon la norme en vigueur a permis de déterminer la teneur massique en BTEX dans l'échantillon de sol.
L'analyse rapide par IR a permis de détecter les BTEX au pic spécifique de 1620 cm⁻¹.
Les résultats sont présentés dans le Tableau 3 ci-dessous.

**[Tableau 3]**

| **Polluants** | **Ech. 5** |
|---|---|
| Teneur massique en BTEX | 6,5 |
| (mg/Kg de matières sèche) | |
| selon norme ISO 22155 | |
| Aire du pic à 1620 cm⁻¹ | 0,23 |

Le Tableau 3 met en évidence qu'il est possible de détecter rapidement la présence des BTEX dans un sol grâce à une analyse par spectroscopie infrarouge d'un échantillon de ce sol.
De plus les aires des pics calculées et présentées dans le Tableau 3 sont en accord avec la teneur massique en BTEX dans le sol déterminée par l'analyse conventionnelle selon la norme en vigueur.

## Revendications

1. Méthode d'analyse d'un matériau d'excavation comprenant les étapes suivantes :
a) analyse par spectroscopie infrarouge d'un échantillon d'un matériau d'excavation dans une gamme de nombres d'onde comprise entre 4000 cm⁻¹ et 500 cm⁻¹ pour obtenir un spectre infrarouge,
b) détermination de la teneur massique en polluants organiques dans ledit échantillon à partir du spectre infrarouge obtenu à l'étape a),
les polluants organiques étant choisis parmi les hydrocarbures totaux, les hydrocarbures aromatiques polycycliques, les composés organiques volatils, les polychlorobiphényles, les BTEX et leurs combinaisons, **caractérisée en ce que** l'étape b) comprend les sous-étapes suivantes :
b11) détection entre 1600 cm⁻¹ et 1700 cm⁻¹ d'une bande d'absorption spécifique aux hydrocarbures totaux dans le spectre infrarouge obtenu à l'étape a),
b21) intégration de la bande d'absorption détectée à l'étape b11) pour calculer une aire, et
b31) comparaison de l'aire obtenue à l'étape b21) avec une courbe de calibration pour déterminer la teneur massique en hydrocarbures totaux du matériau d'excavation.

2. Méthode selon la revendication 1 dans lequel le matériau d'excavation présente un taux de matière sèche de 70% à 100%.

3. Méthode selon la revendication 1 ou la revendication 2 dans lequel, avant l'étape a), le matériau d'excavation subit une étape de broyage pour présenter une granulométrie telle que 35% massique du matériau d'excavation présente un diamètre inférieur à 63 micromètres.

4. Méthode selon l'une quelconques des revendications 1 à 3 dans lequel l'étape b) comprend les sous-étapes suivantes :
b12) détection entre 1400 cm⁻¹ et 1500 cm⁻¹ d'une bande d'absorption spécifiques aux hydrocarbures aromatiques polycycliques dans le spectre infrarouge obtenu à l'étape a),
b22) intégration de la bande d'absorption détectée à l'étape b12) pour calculer une aire, et
b32) comparaison de l'aire obtenue à l'étape b22) avec une courbe de calibration pour déterminer la teneur massique en hydrocarbures aromatiques polycycliques du matériau d'excavation.

5. Méthode selon l'une quelconques des revendications 1 à 4 dans lequel l'étape b) comprend les sous-étapes suivantes :
b13) détection entre 3300 cm⁻¹ et 3400 cm⁻¹ d'une bande d'absorption spécifique aux composés organiques volatils dans le spectre infrarouge obtenu à l'étape a),
b23) intégration de la bande d'absorption détectée à l'étape b13) pour calculer une aire, et
b33) comparaison de l'aire obtenue à l'étape b23) avec une courbe de calibration pour déterminer la teneur massique en composés organiques volatils du matériau d'excavation.

6. Méthode selon l'une quelconques des revendications 1 à 5 dans lequel l'étape b) comprend les sous-étapes suivantes :
b14) détection entre 800 cm⁻¹ et 500 cm⁻¹ d'une ou de deux bandes d'absorption spécifiques aux polychlorobiphényles dans le spectre infrarouge obtenu à l'étape a),
b24) intégration des bandes d'absorption détectées à l'étape b14) pour calculer une aire, et
b34) comparaison de l'aire obtenue à l'étape b24) avec une courbe de calibration pour déterminer la teneur massique en polychlorobiphényles du matériau d'excavation.

7. Méthode selon l'une quelconques des revendications 1 à 6 dans lequel l'étape b) comprend les sous-étapes suivantes :
b15) détection entre 1650 cm⁻¹ et 1600 cm⁻¹ d'une bande d'absorption spécifique aux BTEX dans le spectre infrarouge obtenu à l'étape a),
b25) intégration de la bande d'absorption détectée à l'étape b15) pour calculer une aire, et
b35) comparaison de l'aire obtenue à l'étape b25) avec une courbe de calibration pour déterminer la teneur massique en BTEX du matériau d'excavation.

8. Méthode de sélection d'une filière de retraitement de matériau d'excavation comprenant les étapes suivantes:
c) comparaison de la teneur massique de chaque polluant organique déterminée par la méthode d'analyse telle que définie selon l'une quelconques des revendications 1 à 7 avec une valeur limite fixée,
d) sélection de la filière de traitement selon les critères suivants:
- si la teneur massique de chaque polluant organique contenu dans l'échantillon est inférieure à la valeur limite fixée alors le matériau est considéré comme inerte,
- si la teneur massique d'un polluant organique est supérieure à la valeur limite fixée alors le matériau est considéré comme contaminé et peut être stocké dans des décharges spécialisées ou peut être décontaminé.

9. Méthode de valorisation d'un matériau d'excavation considéré comme inerte selon la méthode de sélection telle que définie à la revendication 8 comprenant une étape de valorisation dudit matériau d'excavation en tant que matériau de construction.

10. Méthode selon la revendication 9 dans lequel le matériau de construction est un remblai ou un granulat.

11. Méthode de stockage d'un matériau d'excavation considéré comme contaminé selon la méthode de sélection telle que définie à la revendication 8 comprenant une étape de stockage dudit matériau d'excavation.

12. Méthode de décontamination d'un matériau d'excavation considéré comme contaminé selon la méthode de sélection telle que définie à la revendication 8 comprenant une étape de décontamination dudit matériau d'excavation.

## Patentansprüche

1. Verfahren zur Analyse von Aushubmaterial, das die folgenden Schritte umfasst:
a) Analyse einer Probe eines Aushubmaterials mittels Infrarotspektroskopie in einem Wellenzahlbereich zwischen 4000 cm⁻¹ und 500 cm⁻¹, um ein Infrarotspektrum zu erhalten,
b) Bestimmung des Massengehalts an organischen Schadstoffen in der Probe aus dem in Schritt a) erhaltenen Infrarotspektrum, wobei die organischen Schadstoffe gewählt sind aus den Gesamtkohlenwasserstoffen, den polyzyklischen aromatischen Kohlenwasserstoffen, den flüchtigen organischen Verbindungen, den polychlorierten Biphenylen, den BTEX und ihren Kombinationen,
**dadurch gekennzeichnet, dass** Schritt b) die folgenden Teilschritte umfasst:
b11) Detektion eines für Gesamtkohlenwasserstoffe spezifischen Absorptionsbereichs im Infrarotspektrum, das in Schritt a) erhalten wurde, zwischen 1600 cm⁻¹ und 1700 cm⁻¹,
b21) Integrieren des in Schritt b11) ermittelten Absorptionsbereichs zur Berechnung einer Fläche, und
b31) Vergleichen der in Schritt b21) erhaltenen Fläche mit einer Kalibrierungskurve zur Bestimmung des Massengehalts an Gesamtkohlenwasserstoffen des Aushubmaterials.

2. Verfahren nach Anspruch 1, wobei das Aushubmaterial einen Trockensubstanzgehalt von 70 % bis 100 % aufweist.

3. Verfahren nach Anspruch 1 oder Anspruch 2, wobei das Aushubmaterial vor Schritt a) einem Zerkleinerungsschritt unterzogen wird, um eine solche Korngröße aufzuweisen, dass 35 Gew.-% des Aushubmaterials einen Durchmesser von weniger als 63 Mikrometern aufweisen.

4. Verfahren nach einem der Ansprüche 1 bis 3, wobei Schritt b) die folgenden Teilschritte umfasst:
b12) Detektion eines für polyzyklische aromatische Kohlenwasserstoffe spezifischen Absorptionsbereichs im Infrarotspektrum, das in Schritt a) erhalten wurde, zwischen 1400 cm⁻¹ und 1500 cm⁻¹,
b22) Integration des in Schritt b12) ermittelten Absorptionsbereichs zur Berechnung einer Fläche, und
b32) Vergleich der in Schritt b22) erhaltenen Fläche mit einer Kalibrierungskurve zur Bestimmung des Massengehalts an polyzyklischen aromatischen Kohlenwasserstoffen im Aushubmaterial.

5. Verfahren nach einem der Ansprüche 1 bis 4, wobei Schritt b) die folgenden Teilschritte umfasst:
b13) Detektion eines für flüchtige organische Verbindungen spezifischen Absorptionsbereichs im Infrarotspektrum, das in Schritt a) erhalten wurde, zwischen 3300 cm⁻¹ und 3400 cm⁻¹,
b23) Integration des in Schritt b13) erfassten Absorptionsbereichs zur Berechnung einer Fläche, und
b33) Vergleich der in Schritt b23) erhaltenen Fläche mit einer Kalibrierungskurve zur Bestimmung des Massengehalts an flüchtigen organischen Verbindungen im Aushubmaterial.

6. Verfahren nach einem der Ansprüche 1 bis 5, wobei Schritt b) die folgenden Teilschritte umfasst:
b14) Detektion einer oder zweier für polychlorierte Biphenyle spezifischer Absorptionsbereiche im Infrarotspektrum, das in Schritt a) erhalten wurde, zwischen 800 cm⁻¹ und 500 cm⁻¹,
b24) Integration der in Schritt b14) ermittelten Absorptionsbereiche zur Berechnung einer Fläche, und
b34) Vergleich der in Schritt b24) erhaltenen Fläche mit einer Kalibrierungskurve zur Bestimmung des Massengehalts an polychlorierten Biphenylen im Aushubmaterial.

7. Verfahren nach einem der Ansprüche 1 bis 6, wobei Schritt b) die folgenden Teilschritte umfasst:
b15) Detektion eines für BTEX spezifischen Absorptionsbereichs im Infrarotspektrum, das in Schritt a) erhalten wurde, zwischen 1650 cm⁻¹ und 1600 cm⁻¹,
b25) Integration des in Schritt b15) ermittelten Absorptionsbereichs zur Berechnung einer Fläche, und
b35) Vergleich der in Schritt b25) erhaltenen Fläche mit einer Kalibrierungskurve zur Bestimmung des Massengehalts an BTEX im Aushubmaterial.

8. Verfahren zur Auswahl eines Weges zur Wiederaufbereitung von Aushubmaterial, das die folgenden Schritte umfasst:
c) Vergleich des Massengehalts jedes organischen Schadstoffs, der durch das Analyseverfahren gemäß einem der Ansprüche 1 bis 7 bestimmt wurde, mit einem festgelegten Grenzwert,
d) Auswahl des Behandlungsweges nach folgenden Kriterien:
- Wenn der Massengehalt jedes in der Probe enthaltenen organischen Schadstoffs unter dem festgelegten Grenzwert liegt, wird das Material als inert betrachtet,
- Wenn der Massengehalt eines organischen Schadstoffs über dem festgelegten Grenzwert liegt, wird das Material als kontaminiert betrachtet und kann in speziellen Deponien gelagert oder dekontaminiert werden.

9. Verfahren zur Verwertung von Aushubmaterial, das gemäß dem in Anspruch 8 definierten Auswahlverfahren als inert angesehen wird, umfassend einen Schritt zur Verwertung des Aushubmaterials als Baumaterial.

10. Verfahren nach Anspruch 9, wobei das Baumaterial ein Füllmaterial oder ein Granulat ist.

11. Verfahren zur Lagerung von Aushubmaterial, das gemäß dem in Anspruch 8 definierten Auswahlverfahren als kontaminiert gilt, umfassend einen Schritt der Lagerung des Aushubmaterials.

12. Verfahren zur Dekontamination von Aushubmaterial, das gemäß dem in Anspruch 8 definierten Auswahlverfahren als kontaminiert gilt, umfassend einen Schritt zur Dekontamination des Aushubmaterials.

## Claims

1. A method for analysing an excavation material comprising the following steps:
a) analysing by infrared spectroscopy a sample of an excavation material in a range of wavenumbers between 4000 cm⁻¹ and 500 cm⁻¹ to obtain an infrared spectrum,
b) determining the mass content of organic pollutants in said sample from the infrared spectrum obtained in step a), the organic pollutants being selected from total hydrocarbons, polycyclic aromatic hydrocarbons, volatile organic compounds, polychlorobiphenyls, BTEXs, and combinations thereof, **characterised in that** step b) comprises the following sub-steps:
b11) detecting between 1600 cm⁻¹ and 1700 cm⁻¹ an absorption band specific to total hydrocarbons in the infrared spectrum obtained in step a),
b21) integrating the absorption band detected in step b11) to calculate an area, and
b31) comparing the area obtained in step b21) with a calibration curve to determine the mass content of total hydrocarbons of the excavation material.

2. The method according to claim 1, wherein the excavation material has a dry matter content of 70% to 100%.

3. The method according to claim 1 or claim 2, wherein, prior to step a), the excavation material undergoes a grinding step to have a grain size such that 35% by mass of the excavation material has a diameter of less than 63 micrometres.

4. The method according to any of claims 1 to 3, wherein step b) comprises the following sub-steps:
b12) detecting between 1400 cm⁻¹ and 1500 cm⁻¹ an absorption band specific to polycyclic aromatic hydrocarbons in the infrared spectrum obtained in step a),
b22) integrating the absorption band detected in step b12) to calculate an area, and
b32) comparing the area obtained in step b22) with a calibration curve to determine the mass content of polycyclic aromatic hydrocarbons of the excavation material.

5. The method according to any of claims 1 to 4, wherein step b) comprises the following sub-steps:
b13) detecting between 3300 cm⁻¹ and 3400 cm⁻¹ an absorption band specific to volatile organic compounds in the infrared spectrum obtained in step a),
b23) integrating the absorption band detected in step b13) to calculate an area, and
b33) comparing the area obtained in step b23) with a calibration curve to determine the mass content of volatile organic compounds of the excavation material.

6. The method according to any of claims 1 to 5, wherein step b) comprises the following sub-steps:
b14) detecting between 800 cm⁻¹ and 500 cm⁻¹ one or two absorption bands specific to polychlorobiphenyls in the infrared spectrum obtained in step a),
b24) integrating the absorption bands detected in step b14) to calculate an area, and
b34) comparing the area obtained in step b24) with a calibration curve to determine the mass content of polychlorobiphenyls of the excavation material.

7. The method according to any of claims 1 to 6, wherein step b) comprises the following sub-steps:
b15) detecting between 1650 cm⁻¹ and 1600 cm⁻¹ an absorption band specific to BTEXs in the infrared spectrum obtained in step a),
b25) integrating the absorption band detected in step b15) to calculate an area, and
b35) comparing the area obtained in step b25) with a calibration curve to determine the mass content of BTEXs of the excavation material.

8. A method for selecting an excavation material reprocessing sector comprising the following steps:
c) comparing the mass content of each organic pollutant determined by the analysis method as defined according to any of claims 1 to 7 with a preset limit value,
d) selecting the processing sector according to the following criteria:
- if the mass content of each organic pollutant contained in the sample is less than the preset limit value, then the material is considered as inert,
- if the mass content of an organic pollutant is higher than the preset limit value, then the material is considered as contaminated and may be stored in specialised landfills or may be decontaminated.

9. A method for reclaiming an excavation material considered as inert according to the selection method as defined in claim 8 comprising a step of reclaiming said excavation material as a construction material.

10. The method according to claim 9, wherein the construction material is backfill or aggregate.

11. A method for storing an excavation material considered as contaminated according to the selection method as defined in claim 8 comprising a step of storing said excavation material.

12. A method for decontaminating an excavation material considered as contaminated according to the selection method as defined in claim 8 comprising a step of decontaminating said excavation material.
